# EUROPÄISCHE PATENTSCHRIFT

(11) **EP 1 443 858 B1**
(45) Veröffentlichungstag und Bekanntmachung des Hinweises auf die Patenterteilung: **09.11.2005**
(21) Anmeldenummer: 02785049.4
(22) Anmeldetag: 05.11.2002
(51) Int. Cl.: A61B 6/14

(54) **Dentales Röntgengerät mit bewegbarer Trägerstruktur**
DENTAL X-RAY DEVICE COMPRISING A MOBILE SUPPORT STRUCTURE
Appareil de radiographie dentaire comprenant une structure de support mobile

(30) Priorität: 06.11.2001 DE 10153979
(43) Veröffentlichungstag der Anmeldung: 11.08.2004
(73) Patentinhaber: Sirona Dental Systems GmbH, 64625 Bensheim (DE)
(72) Erfinder: STÖCKL, Klaus, 64625 Bensheim (DE)
(74) Vertreter: Sommer, Peter
(86) Internationale Anmeldenummer: PCT/DE2002/004108
(87) Internationale Veröffentlichungsnummer: WO 2003/039372

(56) Entgegenhaltungen:
- WO-A-98/32377
- DE-A- 2 120 379
- DE-A- 4 312 640
- US-A- 3 536 913

## Beschreibung

### Technisches Gebiet

Die Erfindung betrifft ein dentales Röntgengerät mit einer bewegbarer Trägerstruktur für ein zur Erstellung von Röntgenaufnahmen zu bewegendes System zur Erstellung von Panoramaaufnahmen, worunter auch Panoramaschichtaufnahmen und transversale Schichtaufnahmen des Kieferbogens zu verstehen sind. Darüber hinaus können mit dem Röntgengerät auch Ceph-Aufnahmen ausgeführt werden. Die bewegbare Trägerstruktur ist mittels eines oder mehrerer Lager auf der feststehenden Trägerstruktur verschiebbar gelagert, wobei das Lager Lagermittel umfasst, die an mindestens einer Trägerstruktur festgelegt sind. Mindestens eine Trägerstruktur weist eine Auflagefläche für die Lagermittel auf. Für die Verschiebung der Trägerstruktur in Richtung parallel zur Auflagefläche sind Verstellmittel vorgesehen. Weiterhin ist ein Antrieb zur Ausführung einer Rotation der Trägerstruktur um eine Achse senkrecht zur Auflagefläche vorhanden.

### Stand der Technik

Aus der EP 0 229 308 ist eine Röntgeneinrichtung bekannt, bei der eine auf einer Trägerplatte mittels Rollen gelagerte Platte in eine definierte Bewegung versetzbar ist, um eine Panorama-Aufnahme (PAN-Aufnahme) zu erstellen. Der Drehbewegung des Röntgenstrahlers und des Bildaufnehmers wird mit einer Kurvenscheibe gekoppelt, so dass bei der Drehung die Platte um eine Längsachse gegenüber der Trägerplatte verschwenkt wird.

Eine weitere Ausführungsform in der EP 0 229 308 zeigt ein Röntgengerät, bei dem ein Verschwenken über ein stabiles Drehlager mit senkrechter Drehachse und die horizontale Bewegung mit Schlittenführungen oder stabilen Doppelgelenkarmen erfolgt. Dabei dreht ein Motor für die Rotationsbewegung über einen Riemen das zu bewegende System aus Röntgenstrahler und des Bildaufnehmer um den Momentanpol, zwei Stellantriebe sorgen für die erforderliche Horizontalbewegung des Momentanpols. Die rotatorische Bewegung und die translatorische Bewegung werden in getrennten Elementen geführt und gelagert.

Aus der WO 98 32 377 ist eine Drehvorrichtung bekannt, welche eine erste feststehende Platte und eine zweite bewegliche Platte aufweist, welche auf der ersten Platte unter Verwendung von mindestens drei Kugeln gelagert ist sowie Mittel zur Bewegung der Platte umfasst. Die sich gegenüber liegenden Oberflächen der Platten weisen jede Vertiefungen auf, deren Anzahl derjenigen der Kugeln entspricht und in welchen die Kugeln rollen können, wobei jede Kugel sowohl in die Vertiefung der feststehenden Platte als auch in die dazu entsprechende Vertiefung der beweglichen Platte eindringt. Die Bahn der Vertiefungen ist derart, dass für jede Lage der beweglichen Platte drei Orte vorhanden sind, wo die Vertiefungen sich genau überdecken und in welchen sich die jeweils entsprechende Kugel befindet, wobei die Kugeln ohne Gleiten abrollen, wenn die bewegliche Platte bewegt wird. Nachteilig hierbei ist, dass nur eine Bahn möglich ist.

Die US 3,536,913 offenbart ein dentales Röntgengerät mit einer bewegbaren und einer feststehenden Trägerstruktur. Die bewegbare Trägerstuktur ist mittels eines Lagers verschiebbar gegenüber der feststehenden Trägerstruktur gelagert. Die bewegbare Trägerstruktur ist so gelagert, dass eine kombinierte Rotations- und Translationsbewegung stattfindet.

Die DE 2 120 379 offenbart eine Röntgenapparatur unter anderem zur Erstellung von dentalen Röntgenaufnahmen, bei der eine Röntgenquelle und ein Röntgenfilm in einer definierten Weise um den Kopf eines Patienten geschwenkt werden können. Die Schwenkbewegung ist dabei durch ein mechanisches System fest vorgegeben.

### Darstellung der Erfindung

Das erfindungsgemäße dentale Röntgengerät umfasst eine erste, feststehende Trägerstruktur und eine zweite Trägerstruktur für ein zur Erstellung von Röntgenaufnahmen zu bewegendes System, welche auf der ersten Trägerstruktur mittels eines oder mehrerer Lager verschiebbar gelagert ist. Das Lager umfasst Lagermittel, die an mindestens einer Trägerstruktur festgelegt sind und mindestens eine Trägerstruktur weist eine Auflagefläche für die Lagermittel auf, wobei ein Antrieb zur Ausführung einer Rotation der Trägerstruktur um eine Achse senkrecht zur Auflagefläche vorgesehen ist. Die Lagermittel sind so ausgebildet, dass sie auf der Auflagefläche in jeder Richtung parallel zur Auflagefläche und zur Ausführung einer Rotation um eine Achse senkrecht zur Auflagefläche bewegbar sind.

Für die Verschiebung der Trägerstruktur in Richtung parallel zur Auflagefläche sind mindestens zwei Verstellmittel vorgesehen, die die bewegbare Trägerstruktur und damit den Momentanpol der durch den Antrieb bewerkstelligten Rotationsbewegung unabhängig von der Rotationsbewegung parallel zur Auflagefläche verschieben.

Der Vorteil der vorliegenden Erfindung besteht darin, die getrennten Lagerungs- und Führungselemente so zusammenzufassen, dass sowohl die Rotations- als auch die Translationsbewegung möglich ist. Durch die auf der Auflagefläche frei bewegbar und damit ungeführt aufliegenden Lagermittel lassen sich drei Freiheitsgrade mit einer einzigen Lagerung verwirklichen, nämlich in horizontaler Richtung rechts/links und vor/zurück sowie eine Drehung um eine senkrechte Achse.

Der Aufbau eines solchen Lagers ist im Vergleich zu bekannten Systemen mit getrennten Elementen für Rotation und Translation sehr viel einfacher und kostengünstiger und erlaubt dennoch eine genaue und leichtgängige Bewegung des Systems auch bei einer großen Masse, insbesondere dann, wenn das Lager Wälzkörper aufweist, die im wesentlichen auf den Lagerflächen abrollen anstatt zu gleiten.

Selbstverständlich ist diese Lagerung auch als Gleitlager ausführbar, erfordert aber wegen des höheren Reibungswiderstandes stärke Stellantriebe.

Eine sehr genaue und sichere Ausführung erreicht man, wenn man die zu bewegende Trägerstruktur zwischen zwei Lagern leicht verspannt. Dies stellt immer noch einen deutlich geringeren Aufwand als bei bisherigen Systemen dar. Darüber hinaus ist die Stabilität und Genauigkeit dieser Lagerung besser als die bisheriger Systeme, da sich dort die Lagerspiele und Instabilitäten der getrennten Lagerungen und Führungen summieren.

Das dentale Röntgengerät kann auch so ausgebildet sein, dass ein Röntgenstrahler an einer ersten beweglichen Trägerstruktur und der Röntgenbildaufnehmer an einer zweiten beweglichen Trägerstruktur befestigt ist, wobei sowohl die den Röntgenstrahler tragende Trägerstruktur als auch die den Röntgenbildaufnehmer tragende Trägerstruktur gegenüber der feststehenden Trägerstruktur frei beweglich sind.

Die Stellantriebe für die Horizontalbewegung können direkt auf die Rotationsachse des Systems wirken oder indirekt über ein an die zu bewegende Trägerstruktur angekoppeltes Element, das dem Mittelpunkt folgt, aber nicht die Rotation der Trägerstruktur mit ausführt.

Weitere Ausgestaltungen der Erfindung sind in den Unteransprüchen beschrieben.

### Kurzbeschreibung der Zeichnung

In der Zeichnung ist ein Ausführungsbeispiel der Erfindung dargestellt. Es zeigt die
- Fig. 1: eine Prinzipskizze von einem dentalen Röntgengerät mit beweglicher Trägerstruktur;
- Fig. 2: die Bewegungsrichtungen der beweglichen Trägerstruktur;
- Fig. 3a: ein erstes Lager, mit welchem die erforderlichen Bewegungen der Trägerstruktur ausführbar sind;
- Fig. 3b: das Lager als drehbar gelagerte Schlepprolle im Detail;
- Fig. 4a: einen Aufbau zur Bewegung der Trägerstruktur;
- Fig. 4b: eine Frontalsicht auf die Schemakonstruktion aus Fig. 4a;
- Fig. 5a: eine andere Ausführung des Lagers, welches in die Trägerstruktur 21 eingelassen ist, wobei sich die Auflagefläche an der Trägerstruktur befindet;
- Fig. 5b: das Lager aus Fig. 5a im Detail;
- Fig. 6a: eine weitere Ausführung einer zwischen zwei Lager eingespannten Trägerstruktur;
- Fig. 6b: einen Kugelkäfig des Lagers aus Fig. 6a.

### Ausführungsbeispiele der Erfindung

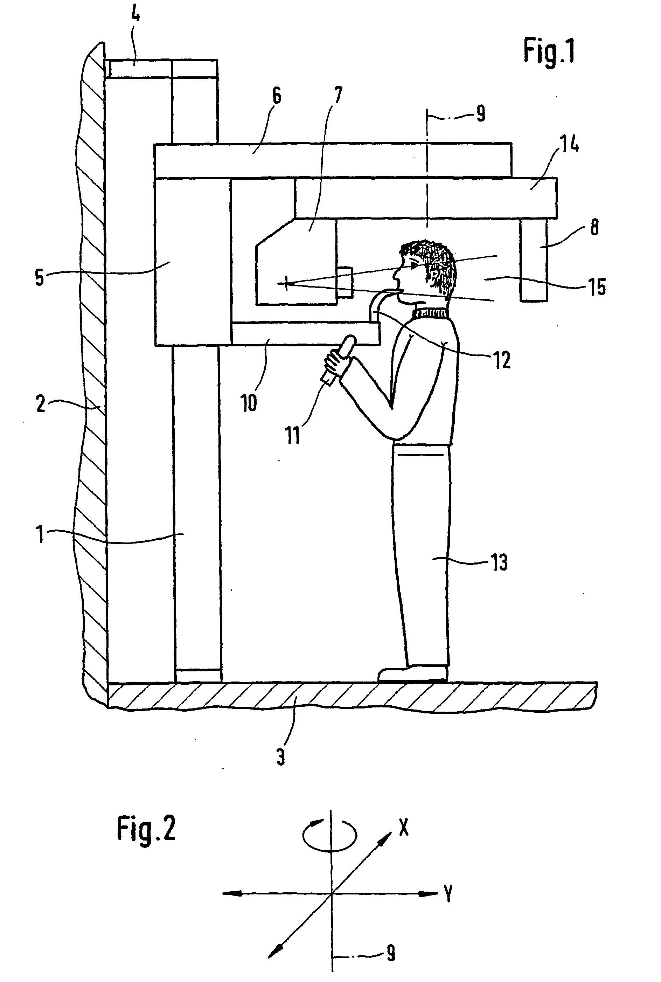

Das in Fig. 1 dargestellte Röntgengerät weist eine vertikale Trägerstruktur 1 auf, die in einem Abstand zu einer Wand 2 verläuft und an Ihrem unteren Ende auf einem Boden 3 aufsitzt. Am oberen Ende der Trägerstruktur 1 ist diese über einen Abstandshalter 4 an der Wand 2 befestigt. An dieser vertikalen Trägerstruktur 1, auch als Säule bezeichnet, ist eine horizontale Trägerstruktur 5 höhenverstellbar geführt.

Der detaillierte Aufbau eines derartigen Röntgengerätes ist in der EP 0 229 308 A1 ausführlich beschrieben, ebenso die Betriebsweise zur Erstellung von Röntgenaufnahmen. Die dort offenbarten Ausführungen werden voll umfänglich in diese Anmeldung mit einbezogen.

An der horizontalen Trägerstruktur 5 ist ein Ausleger 6 vorgesehen, an dem ein Röntgenstrahler 7 und ein Bildaufnehmer 8 um eine Längsachse drehbar befestigt ist.

Zur Positionierung des Patienten sind an der horizontalen Trägerstruktur darüber hinaus an einem Haltearm 10 Haltegriffe 11 und ein Aufbiss 12 vorgesehen, mittels welcher ein Patient 13 während der gemeinsamen Drehung des Röntgenstrahlers 7 und des Bildaufnehmers 8 um die Achse 9 in seiner räumlichen Lage fixiert wird.

Der Röntgenstrahler 7 und der Bildaufnehmer 8 sind durch eine Trägerstruktur 14 miteinander verbunden, so dass ein von dem Röntgenstrahler 7 ausgehende Strahlenkegel 15 auf den entsprechend ausgerichteten Bildaufnehmer trifft.

Mit der vorliegenden Erfindung wird dieser aus dem Stand der Technik bekannte Grundaufbau eines Röntgengerätes zur Erstellung dentaler Panoramaschichtaufnahmen bezüglich der Bewegung des Röntgenstrahlers 7 und des Bildaufnehmers 8 bezüglich der horizontalen Trägerstruktur 5 weiterentwickelt, so dass die in Fig. 2 dargestellten Bewegungsrichtungen, nämlich die Rotation um die Längsachse 9 sowie eine Verschiebung in x- und y-Richtung gegenüber der feststehenden horizontalen Trägerstruktur möglich ist.

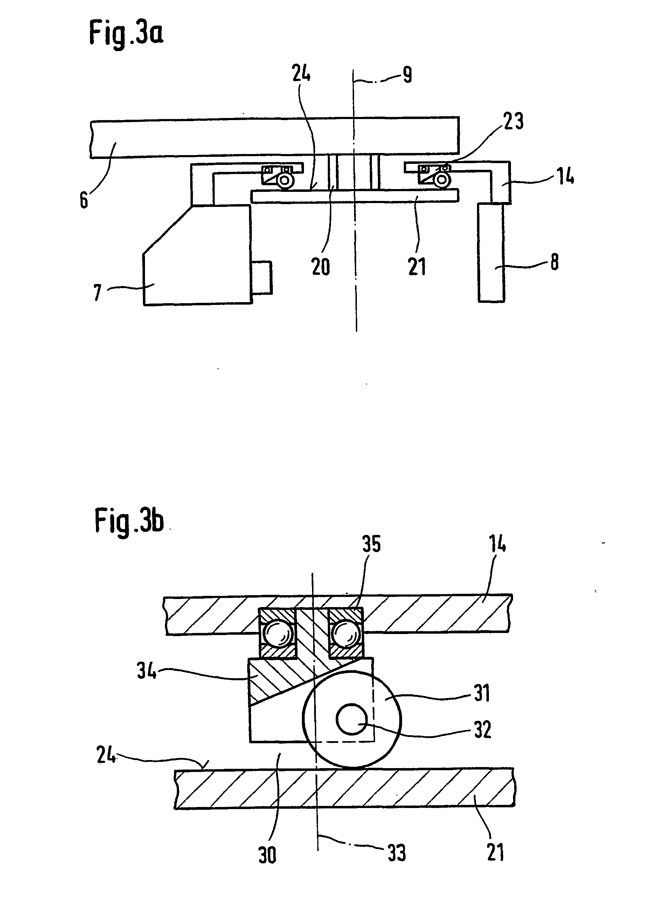

In Fig. 3a ist eine erste Anordnung gezeigt, mit welcher die erforderlichen Bewegungen ausgeführt werden können. An dem Haltearm 6 ist eine Trägerstruktur 21 in einem über eine Abstandshülse 22 hergestellten Abstand befestigt. In dem Zwischenraum zwischen der feststehenden Trägerstruktur 21 und dem Haltearm 6 ist die zweite Trägerstruktur 14 mit dem daran befestigten Röntgenstrahler 7 und diametral gegenüberliegend dem Bildaufnehmer 8 eingebracht und sitzt über ein Wälzlager 23 in Form von Schlepprollen auf einer Auflagefläche 24 der Trägerstruktur 21 auf. Die Schlepprollen sind auf der Auflagefläche 24 frei beweglich.

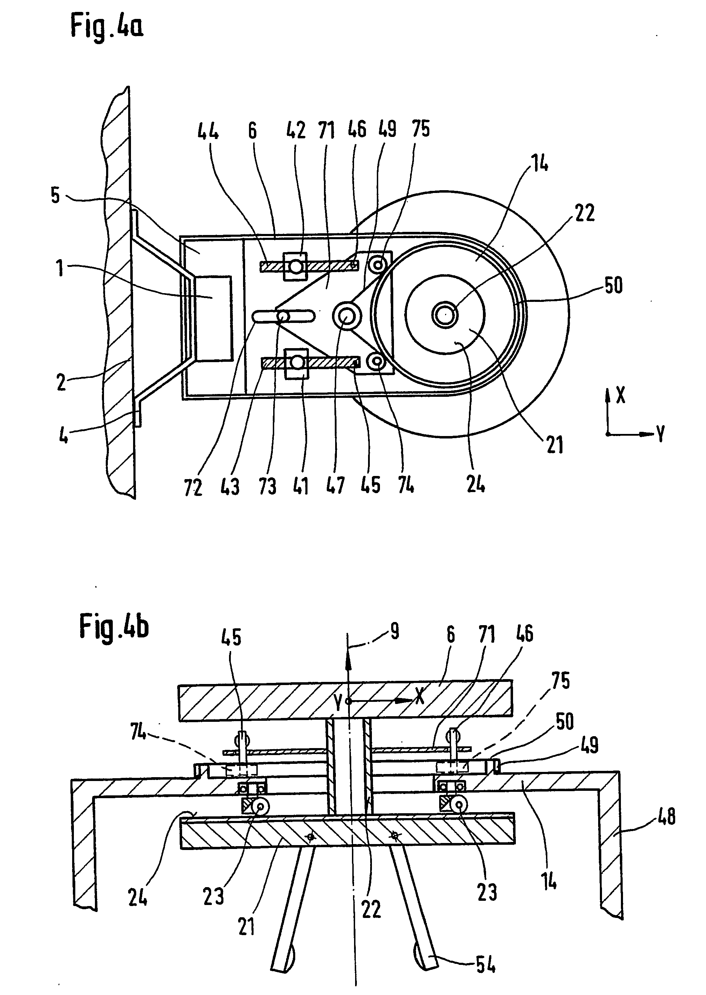

Der Antrieb für die Erzeugung der Bewegung sowie die hierfür erforderliche konstruktive Ausgestaltung wird in den Fig. 4a, 4b erläutert.

In Fig. 3b ist das Lager 23 in der konkreten Ausführung als drehbar gelagerte Schlepprolle im Detail gezeigt. Die Schlepprolle besteht aus einem zylindrischen Wälzkörper 31, der um eine Achse 32 drehbar gelagert ist, wobei die Achse 32 zu einer Hochachse 33 als Drehachse des den Wälzkörper 31 und die Achse 32 aufnehmenden Rollengehäuses 34 beabstandet ist. Das Rollengehäuse 34 ist über ein Kugellager 35 mit der Trägerstruktur 14 verbunden. Derartige Schlepprollen 30 entsprechen dem Stand der Technik und es ist bekannt, dass aufgrund des Abstandes der Achse 32 von der Hochachse 33 der Wälzkörper 31 stets der aufgezwungen Bewegungsrichtung nachläuft. Der Wälzkörper kann ein Kugellager sein, dessen Außenring verstärkt ist und vorzugsweise eine ballige Kontur aufweist. In diesem Fall lässt sich vermeiden dass die Ausrichtung des Wälzkörpers 31 durch Gleiten erfolgt.

In Fig. 4a ist ein möglicher Aufbau zur Bewegung der Trägerstruktur 14 gezeigt. Ausgehend von der vertikalen Trägerstruktur 1, welche über den Abstandshalter 4 an der Wand 2 befestigt ist und dem daran befestigten horizontalen Träger 5 sind an dem Arm 6 Stellantriebe 41, 42 vorgesehen, welche über Spindeln 43,44 auf zwei mit der Trägerstruktur 71 verbundene Führungszapfen 45,46 einwirken. Durch Verstellen der an dem Haltearm 6 drehbar gelagerten Spindeln 43,44 mittels der Stellantriebe 41, 42 lässt sich die Trägerstruktur 71 in x- und y-Richtung verschieben.

Um ein unkontrolliertes Verschwenken der Trägerstruktur 71 zu verhindern ist eine weitere Führung erforderlich. Zu diesem Zweck ist die Trägerstruktur 71 in Form eines Dreiecks ausgebildet. Weiterhin ist ein in einem Langloch 72 der Trägerstruktur 6 in y-Richtung verschiebbar geführter Führungszapfen 73 vorgesehen. Der Führungszapfen 73 ist in der Trägerstruktur 6 so geführt, dass eine Verdrehung der Trägerstruktur 71 um eine in die Zeichenebene hinein verlaufende Hochachse und eine Translation in y-Richtung möglich ist, eine Bewegung in X-Richtung aber unterbleibt.

Über einen Antrieb 47 wird eine Drehbewegung auf die Trägerstruktur 14 übertragen, an welchem der nicht dargestellte Röntgenstrahler und der Bildaufnehmer befestigt ist. Die Drehbewegung wird über einen Riemen 49 übertragen, der auf einem Vorsprung 50 der Struktur 14 läuft. Alternativ ist auch ein Zahnradantrieb vorstellbar. Die Trägerstruktur 14 ist über den Antrieb 47 und weitere Anruckrollen 75, mit der Trägerstruktur 71 verbunden.

Durch das Zusammenspiel von Antrieb 47 und Anpressrollen 74,75 mit dem Vorsprung 50 an der Trägerstruktur 14 ist die Trägerstruktur 14 an der Trägerstruktur 71 in horizontaler Richtung geführt und dabei um eine Hochachse 9 oder parallel dazu drehbar.

Wird die Trägerstruktur 71 parallel zur Auflagefläche 24 der Trägerstruktur 21 mittels der Stellantriebe 41, 42 in x- und y-Richtung verschoben, so bewegt sich die Trägerstruktur 14 mit. Die Trägerstruktur 14 ist gegenüber der Trägerstruktur 71 drehbar ausgebildet und stützt sich bei allen Bewegungen auf der Trägerstruktur 21 (Fig. 4b) ab.

In Fig. 4b ist eine Frontalsicht auf die Schemakonstruktion aus Fig. 4a dargestellt. Ausgehend von dem Haltearm 6, an dem die Trägerstruktur 21 über eine Hülse 22 in einem Abstand befestigt ist, ist die Trägerstruktur 14 mit der außenliegenden Struktur 48 gezeigt. Die Trägerstruktur 14 liegt über die Lager 23 auf der Auflagefläche 24 der Trägerstruktur 21 auf und kann über die mit der Trägerstruktur 71 verbundenen Führungszapfen 45,46, an welchen die Stellantriebe angreifen, in x- und y-Richtung parallel zur Auflagefläche 24 der Trägerstruktur 21 bewegt werden. Bei den Lagern kann es sich um Gleitlager oder um Wälzlager handeln, die auf einer verschleißfesten Auflagefläche 24 aufliegen. Die Auflagefläche 24 ist als von der Trägerstruktur 21 getrenntes Bauteil ausgebildet, nämlich als gehärtete Stahlscheibe auf einem Aluminiumträger mit eigentlich weicher Oberfläche, um Gewicht und Kosten zu sparen.

Die Drehung der Trägerstruktur 14 wird über den als Riemenscheibe wirkenden Vorsprung 50 und den auf der Außerseite des Vorsprungs 50 laufenden Antriebsriemen 49 hergestellt. Der Antrieb 47 kann dabei auf der Trägerstruktur 71 fest montiert sein.

Die elektromotorische Verstellung des Systems entspricht den aus der DE 43 12 640 A1 oder der DE 44 40 376 A1 bekannten Systemen, wo ein Röntgenstrahler und ein Bildaufnehmer um einen Momentanpol drehbar angeordnet sind und der Momentanpol während der Rotation in der horizontalen Ebene verschiebbar ist.

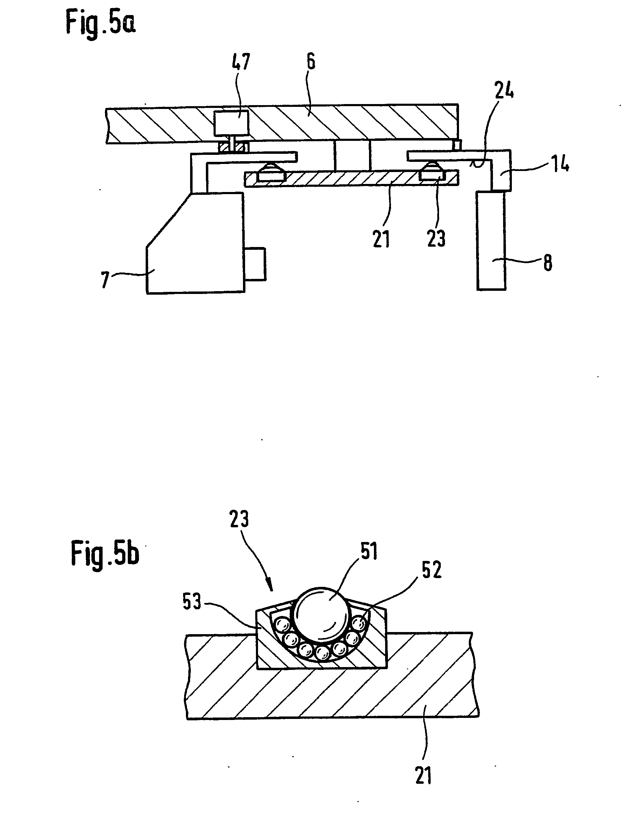

In Fig. 5a ist eine andere Ausführung des Lagers 23 dargestellt. Das Lager 23 ist in die Trägerstruktur 21 eingelassen und die Auflagefläche 24 befindet sich an der Trägerstruktur 14. Der Antrieb 47 für die Drehbewegung des Röntgenstrahlers 7 bzw. des Bildaufnehmers 8 ist an der nicht gezeigten Trägerstruktur 71 befestigt.

In Fig. 5b ist das Lager 23 im Detail gezeigt. Als Lagermittel ist eine Kugel 51 vorgesehen, welche wiederum von kleinen Wälzkörpern in Form von Kugeln 52 in einem Gehäuse 53 gehalten ist. Das Gehäuse 53 ist in die Trägerstruktur 21 eingefügt und in seiner Lage festgelegt.

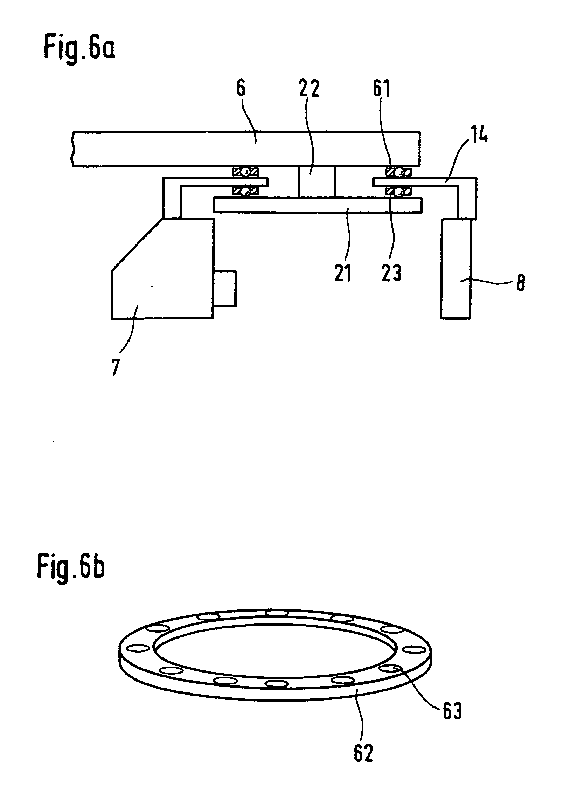

In den Fig. 6a,6b ist eine weitere Ausführung des Lagers 23 gezeigt, wobei hier die Besonderheit neben dem verwendeten Lager darin besteht, dass die Trägerstruktur 14 sowohl zur Trägerstruktur 21 hin als auch zum Haltearm 6 hin über Lager 23,61 abgestützt ist. Die Lager 23,61 sind dabei leicht verspannt, so dass die Trägerstruktur 14 selbst dann spielfrei geführt wird, wenn der Röntgenstrahler 7 und der Bildaufnehmer mit den dazugehörigen Systemen eine unterschiedliche Masse aufweisen. Ein Verkippen der Trägerstruktur 14 ist somit ausgeschlossen.

In Fig. 6b ist ein Käfig 62 für Wälzkörper in Form von Kugeln gezeigt, wobei der Käfig Durchbrechungen 63 für die Aufnahme von Kugeln aufweist.

In dem Ausführungsbeispiel weist die Trägerstruktur 21 eine Auflagefläche 24 auf, auf der die Kugeln des Lagers 23 abrollen, wobei die Kugeln des Lagers 23 auch auf einer Auflagefläche der Trägerstruktur 14 abrollen. Die Trägerstruktur 14 ist darüber hinaus mit einer weiteren Auflagefläche für die Wälzkörper des Lagers 61 zum Haltearm 6 hin versehen, wobei auch der Haltearm 6 eine Auflagefläche für die Wälzkörper des Lagers 61 aufweist. Insgesamt werden zwei Kugelkäfige und vier Auflageflächen als Anlaufscheiben für die Wälzkörper verwendet.

Das Lager kann aber auch mit nur einem Lagerkäfig und zwei Anlaufscheiben ausgeführt werden. Es ist dann dafür Sorge zu tragen, dass der Schwerpunkt des zu bewegenden Teils innerhalb des von den Lagern aufgespannten Polygons liegt, um ein Kippen des Systems zu verhindern.

Die Vorspannung des Lagers wird über die Verbindungshülse 22 eingestellt. Die beidseitige Lagerung ist dabei auch bei Verwendung von Kugelrollen oder Gelenkrollen gemäß Fig 3a, b und Fig. 5a, b oder bei Gleitlagern möglich.

Bei der Trägerstruktur 21 handelt es sich um einen Ring, ebenso bei der Trägerstruktur 14, wobei die Breite des Ringes so bemessen ist, dass der gewünschte Bewegungsablauf von 50 bis 250 mm in x- und/oder y-Richtung möglich ist.

Durch die Verbindungshülse 22 hindurch können sich von dem Haltearm 6 ausgehend Kopffixierungen erstrecken, welche durch die Bewegung der Trägerstruktur 14 nicht beeinflusst werden. Die Kopffixierungen 54 gemäß Fig. 4b sind hingegen direkt an der Trägerstruktur 21 befestigt.

## Patentansprüche

1. Dentales Röntgengerät mit einer bewegbarer Trägerstruktur (14) für ein zur Erstellung von Röntgenaufnahmen zu bewegendes System, umfassend weiterhin eine feststehende Trägerstruktur (21), wobei die bewegbare Trägerstruktur (14) auf der feststehenden Trägerstruktur (21) mittels eines oder mehrerer Lager (23) verschiebbar gelagert ist, wobei das Lager (23) Lagermittel umfasst, die an mindestens einer Trägerstruktur (14,21) festgelegt sind und mindestens eine Trägerstruktur (14,21) eine Auflagefläche (24) für die Lagermittel aufweist, wobei die Lagermittel so ausgebildet sind, dass sie auf der Auflagefläche (24) in jeder Richtung parallel zur Auflagefläche (24) und zur Ausführung einer Rotation um eine Achse senkrecht zur Auflagefläche (24) bewegbar sind, und wobei ein Antrieb (47) zur Ausführung einer Rotation der bewegbaren Trägerstruktur (14) um eine Achse senkrecht zur Auflagefläche (24) vorgesehen ist,
**dadurch gekennzeichnet, dass** für die Verschiebung der bewegbaren Trägerstruktur (14) in Richtung parallel zur Auflagefläche (24) mindestens zwei Verstellmittel (41, 42) vorgesehen sind, die dazu geeignet sind die bewegbare Trägerstruktur (14) und damit den Momentanpol der durch den Antrieb (47) bewerkstelligten Rotationsbewegung unabhängig von der Rotationsbewegung parallel zur Auflagefläche (24) zu verschieben.

2. Dentales Röntgengerät nach Anspruch 1, **dadurch gekennzeichnet, dass** die Lagermittel zwangsgeführte Wälzkörper umfassen.

3. Dentales Röntgengerät nach Anspruch 2, **dadurch gekennzeichnet, dass** als Lagermittel drehbar gelagerte Schlepprollen (30) vorgesehen sind.

4. Dentales Röntgengerät nach Anspruch 2, **dadurch gekennzeichnet, dass** als Lagermittel wälzkörpergelagerte Kugelrollen (51) vorgesehen sind.

5. Dentales Röntgengerät nach Anspruch 2, **dadurch gekennzeichnet, dass** als Lagermittel in einem Käfig (62) zueinander beabstandet angeordnete Wälzkörper, insbesondere in Form von Kugeln vorgesehen sind.

6. Dentales Röntgengerät nach einem der Ansprüche 1 bis 5, **dadurch gekennzeichnet, dass** die zu bewegende Trägerstruktur (14) zwischen zwei Lagern (23,61) gegenüber der Trägerstruktur (21) und dem Haltearm (6) verspannt ist.

7. Dentales Röntgengerät nach einem der Ansprüche 1 bis 6, **dadurch gekennzeichnet, dass** der horizontale Verstellweg auf der Auflagefläche (24) in allen Richtungen zwischen 50 und 250 mm beträgt.

8. Dentales Röntgengerät nach einem der Ansprüche 1 bis 7, **dadurch gekennzeichnet, dass** an einer der Trägerstrukturen (14, 21) eine Kopffixierung (54) vorgesehen ist.

9. Dentales Röntgengerät nach einem der Ansprüche 1 bis 8, **dadurch gekennzeichnet, dass** ein Röntgenstrahler (7) an einer ersten beweglichen Trägerstruktur und der Röntgenbildaufnehmer (8) an einer zweiten beweglichen Trägerstruktur befestigt ist, wobei sowohl die den Röntgenstrahler (7) tragende Trägerstruktur als auch die den Röntgenbildaufnehmer (8) tragende Trägerstruktur gegenüber der feststehenden Trägerstruktur (21) frei beweglich sind.

10. Dentales Röntgengerät nach einem der Ansprüche 1 bis 9, wobei ein Antrieb (47) zur Erzeugung einer Rotationsbewegung des zu bewegenden Systems um einen Momentanpol und mindestens zwei Stellantriebe (41,42) für die Horizontalbewegung des Momentanpols vorgesehen ist, **dadurch gekennzeichnet, dass** die Stellantriebe (41,42) für die Horizontalbewegung direkt auf die Rotationsachse des Systems wirken oder indirekt über ein angekoppeltes Element (71), das dem Mittelpunkt folgt, aber die Rotation nicht mit ausführt.

## Claims

1. A dental X-ray device having a movable supporting structure (14) for a system adapted to be movable for the purpose of making X-ray images, and having a stationary supporting structure (21) on which said movable supporting structure (14) is displaceably mounted by means of at least one bearing (23), which at least one bearing (23) has bearing elements that are secured to at least one supporting structure (14, 21), and at least one supporting structure (14, 21) has a bearing face (24) for said bearing elements, which bearing elements are adapted for movement on said bearing face (24) in any direction parallel to said bearing face (24) and for rotation about an axis perpendicular to said bearing face (24), and a drive (47) is provided to effect rotation of said movable supporting structure (14) about an axis perpendicular to said bearing face (24), **characterized in that** for displacement of said movable supporting structure (14) in a direction parallel to said bearing face (24) there are provided at least two actuators (41, 42) which are adapted to displace said movable supporting structure (14) and thus the instant center of the rotational movement caused by said drive (47) independently of the rotational movement parallel to said bearing face (24).

2. A dental X-ray device as defined in claim 1, **characterized in that** said bearing elements comprise positively guided rolling elements.

3. A dental X-ray device as defined in claim 2, **characterized in that** said bearing elements are pivotally mounted drag rollers (30).

4. A dental X-ray device as defined in claim 2, **characterized in that** bearing elements are provided which are spherical rollers mounted on rolling elements (51).

5. A dental X-ray device as defined in claim 2, **characterized in that** bearing elements are provided which are rolling elements, particularly in the form of balls, which are disposed in a cage (62) at spaced intervals.

6. A dental X-ray device as defined in any one of claims 1 to 5, **characterized in that** said movable supporting structure (14) is clamped between two bearings (23, 61), one on said supporting structure (21) and the other on said holding arm (6).

7. A dental X-ray device as defined in any one of claims 1 to 6, **characterized in that** the horizontal adjustment range on said bearing face (24) in all directions is from 50 to 250 mm.

8. A dental X-ray device as defined in any one of claims 1 to 7, **characterized in that** a head holder (54) is provided on one of the supporting structures (14, 21).

9. A dental X-ray device as defined in any one of claims 1 to 8, **characterized in that** an X-ray unit (7) is mounted on a first movable supporting structure and an X-ray image detector (8) is mounted on a second movable supporting structure, both the supporting structure carrying said X-ray unit (7) and the supporting structure carrying said X-ray image detector (8) being freely movable relatively to said stationary supporting structure (21).

10. A dental X-ray device as defined in any one of claims 1 to 9, wherein a drive (47) for effecting rotational movement of the movable system about an instant center and at least two actuators (41, 42) for effecting horizontal movement of the instant center are provided, **characterized in that** said actuators (41, 42) for effecting said horizontal movement act directly on the rotation axis of said system or indirectly via a coupled element (71) which follows the instant center but does not follow the rotational movement.

## Revendications

1. Appareil de radiographie dentaire comprenant une structure de support mobile (14) pour un système à déplacer en vue de créer des radiographies, comprenant en outre une structure de support fixe (21), la structure de support mobile (14) étant montée de manière à pouvoir glisser sur la structure de support fixe (21) au moyen d'un ou de plusieurs paliers (23), le palier (23) comprenant des moyens de palier qui sont fixés sur au moins une structure de support (14, 21) et au moins une structure de support (14, 21) présentant une surface d'appui (24) pour les moyens de palier, les moyens de palier étant réalisés de telle sorte qu'ils puissent être déplacés sur la surface d'appui (24) dans chaque direction parallèlement à la surface d'appui (24) et pour réaliser une rotation autour d'un axe perpendiculaire à la surface d'appui (24), et où un entraînement (47) pour la réalisation d'une rotation de la structure de support mobile (14) autour d'un axe perpendiculaire à la surface d'appui (24) est prévu,
**caractérisé en ce que**
pour le glissement de la structure de support mobile (14) dans la direction parallèlement à la surface d'appui (24), au moins deux moyens de réglage (41, 42) sont prévus, lesquels sont prévus pour déplacer la structure de support mobile (14) et de ce fait le pôle temporaire du mouvement de rotation provoqué par l'entraînement (47) indépendamment du mouvement de rotation parallèlement à la surface d'appui (24).

2. Appareil de radiographie dentaire selon la revendication 1, **caractérisé en ce que** les moyens de palier comprennent des corps de roulement guidés par force.

3. Appareil de radiographie dentaire selon la revendication 2, **caractérisé en ce que** l'on prévoit en tant que moyens de palier des rouleaux d'entraînement (30) montés à rotation.

4. Appareil de radiographie dentaire selon la revendication 2, **caractérisé en ce que** l'on prévoit en tant que moyens de palier des rouleaux sphériques (51) montés sur des corps de roulement.

5. Appareil de radiographie dentaire selon la revendication 2, **caractérisé en ce que** l'on prévoit en tant que moyens de palier des corps de roulement espacés les uns des autres dans une cage (62), notamment en forme de billes.

6. Appareil de radiographie dentaire selon l'une quelconque des revendications 1 à 5, **caractérisé en ce que** la structure de support à déplacer (14) est serrée entre deux paliers (23, 61) contre la structure de support (21) et le bras de fixation (6).

7. Appareil de radiographie dentaire selon l'une quelconque des revendications 1 à 6, **caractérisé en ce que** la course de réglage horizontale sur la surface d'appui (24) est comprise entre 50 et 250 mm dans toutes les directions.

8. Appareil de radiographie dentaire selon l'une quelconque des revendications 1 à 7, **caractérisé en ce que** l'on prévoit une fixation pour la tête (54) sur l'une des structures de support (14, 21).

9. Appareil de radiographie dentaire selon l'une quelconque des revendications 1 à 8, **caractérisé en ce qu'**un émetteur de rayons X (7) est fixé sur une première structure de support mobile et **en ce que** le dispositif de radiographie (8) est fixé sur une deuxième structure de support mobile, la structure de support portant l'émetteur de rayons X (7) et la structure de support portant le dispositif de radiographie (8) étant librement mobiles par rapport à la structure de support fixe (21).

10. Appareil de radiographie dentaire selon l'une quelconque des revendications 1 à 9, dans lequel un entraînement (47) pour la production d'un mouvement de rotation du système à déplacer autour d'un pôle temporaire et au moins deux entraînements de réglage (41, 42) pour le déplacement horizontal du pôle temporaire sont prévus, **caractérisé en ce que** les entraînements de réglage (41, 42) pour le mouvement horizontal agissent directement sur l'axe de rotation du système ou indirectement par le biais d'un élément accouplé (71), qui suit le centre, mais qui n'effectue pas la rotation.
